# EUROPEAN PATENT SPECIFICATION

(11) **EP 0 252 146 B1**
(45) Date of publication and mention of the grant of the patent: **25.05.1994**
(21) Application number: 87900968.6
(22) Date of filing: 09.01.1987
(51) Int. Cl.: C07K 15/06, C12N 15/00, C12Q 1/68

(54) **NUCLEAR FACTORS ASSOCIATED WITH TRANSCRIPTIONAL REGULATION**
NUKLEARE FAKTOREN, DIE MIT DER REGULIERUNG DER TRANSKRIPTION ASSOZIIERT SIND
FACTEURS NUCLEAIRES ASSOCIES A LA REGULATION DE LA TRANSCRIPTION

(30) Priority: 09.01.1986 US 817441
(43) Date of publication of application: 13.01.1988
(60) Divisional application: 93110106.7
(73) Proprietor: MASSACHUSETTS INSTITUTE OF TECHNOLOGY, Cambridge, MA 02139 (US); WHITEHEAD INSTITUTE FOR BIOMEDICAL RESEARCH, Cambridge, MA 02142 (US)
(72) Inventor: BALTIMORE, David, Cambridge, MA 02138 (US); SEN, Ranjan, Somerville, MA 02144 (US); SHARP, Phillip, A., Newton, MA 02158 (US); SINGH, Harinder, Cambridge, MA 02139 (US); STAUDT, Louis, A., Watertown, MA 02142 (US)
(74) Representative: Holdcroft, James Gerald, Dr.
(86) International application number: US8700086
(87) International publication number: WO8704170

(56) References cited:
- Science, vol. 227, no.4684, 18 January 1985, M. Mercola et al "Immunoglobulin heavy-chain enhancer requires one or more tissue-specific factors", pages 266-270
- Nature, vol. 319, no.6049, 9 January 1986, H. Singh et al "A nuclear factor that binds to a conserved sequence motif in transcriptional control elements of immunoglobulin genes", pages 154-158

## Description

### Field of the Invention

This invention is in the field of molecular biology.

### Background of the Invention

Trans-acting factors that mediate B cell specific transcription of immunoglobulin (Ig) genes have been postulated based on an analysis of the expression of exogenously introduced Ig gene recombinants in lymphoid and non-lymphoid cells, see M.Mercola et al., Science, Vol. 227, pp 266-270 (1985). Two B cell-specific, cis-acting transcriptional regulatory elements have been identified. One element is located in the intron between the variable and constant regions of both heavy and Kappa light chain genes and acts as a transcriptional enhancer. The second element is found upstream of both heavy chain and Kappa light chain gene promoters. This element directs lymphoid-specific transcription even in the presence of viral enhancers.

Mouse and human light chain promoters contain the octamer sequence ATTTGCAT approximately 70 base pairs upstream from the site of initiation. Heavy chain gene promoters contain the identical sequence in inverted orientation, ATGCAAAT, at the same Position. This element appears to be required for the efficient utilization of Ig promoters in B cells. The high degree of sequence and positional conservation of this element as well as its apparent functional requirement suggests its interaction with a sequence-specific transcription factor but no such factor has been identified.

### Disclosure of the Invention

This invention pertains to human lymphoid-cell nuclear factors which bind to gene elements associated with regulation of the transcription of Ig genes as defined in the claims appended hereto. The factors are implicated in the regulation of transcription of Ig genes.

Four different factors which bind to transciptional regulatory DNA elements of Ig genes were identified and isolated in nuclear extracts of lymphoid cells. Two of the factors (IgNF-A and E) are constitutive; two (IgNF-B and Kappa-3) are lymphoid cell specific. Each factor is described in detail below.

### IgNF-A

IgNF-A binds to DNA sequences in the upstream regions of both the murine heavy and kappa light chain gene promoters and also to the murine heavy chain gene enhancer. The binding is sequence specific and is probably mediated by a highly conserved sequence motif, ATTTGCAT, present in all three transcriptional elements. A factor with binding specificity similar to IgNF-A is also present in human HeLa cells indicating that IgNF-A may not be tissue specific.

### E

E is a constitutive factor which binds to the light and heavy chain enhancers.

### IgNF-B

IgNF-B exhibits the same sequence-specificity as IgNF-A; it binds to upstream regions of murine heavy and kappa light chain gene promoters and to murine heavy chain gene enhancer. This factor, however, is lymphoid specific; it is restricted to B and T cells.

### Kappa-3

Kappa-3 binds exclusively to the Kappa light chain gene enhancer. It is specific to B-cells and also appears to be B-cell stage specific.

The factors were identified and isolated by means of a modified DNA binding assay. The assay has general applicability for analysis of protein DNA interactions in eukaryotic cells. In performing the assay, DNA probes embodying the relevant DNA elements or segments thereof are incubated with cellular nuclear extracts. The incubation is performed under conditions which allows the formation of protein-DNA complexes. Protein-DNA complexes are resolved from uncomplexed DNA by electrophoresis through polyacrylamide gels in low ionic strength buffers. In order to minimize formation of nonspecific complex a competitor DNA species can be added to the incubation mixture of the extract and DNA probe. In the present work with eukaryotic cells the addition of alternating copolymer duplex poly(dI-dC).poly(dI-dC) as a competitor DNA species provided for an enhancement of sensitivity in the detection of nuclear protein-DNA complexes and facilitated detection of the regulatory factors described herein.

### Brief Description of the Drawings

Figure 1a is a schematic depiction of the 5' region of the MOPC 41 V_{Kappa} gene segment; Figure 1b is an autoradiograph of gel electrophoresis DNA binding assays with the SfaNI-SfaNI Kappa promoter fragment of the MOPC 41 V_{Kappa} gene; and Figure 1c is an autoradiograph of gel electrophoresis DNA binding assay with overlapping Kappa promoter fragments.

Figure 2 show autoradiographs of binding competition analysis in nuclear extracts of human (a) EW and (b) HeLa nuclear extracts.

Figure 3 shows the results of DNase I foot printing analysis of factor-DNA complexes.

Figure 4a shows the nucleotide sequences of actual and putative binding sites of IgNF-A; Figure 4b is an autoradiograph of binding assays with various DNA probes of three Ig transcriptional control elements.

Figure 5a shows the DNA sequence of the promoter region of MODC41; Figure 5b shows an autoradiograph of RNA transcript generalized in whole cell extracts made from human B lymphoma cell lines RAMOS and EW and from HeLa cells from the indicated templates.

Figure 6 shows an autoradiogarph of RNA transcripts from templated containing an upstream deletion.

Figure 7 is a radioautograph of the sending of B cell nuclear extract to the MOPC-41 K promoter region showing the IgNF-A and IgNF-B complexes.

Figure 8 shows the binding of T cell and nonlymphoid cell nuclear extracts to the MOPC-41K promoter region.

Figure 9a shows a restriction map of the u - Enhancer; Figure 9b shows an autoradiograph binding assay carried out with u Enhancer fragments.

Figure 10a shows a restriction map of the u300 fragment; Figure 10b shows complexes formed by various subfragments of u300; Figure 10c and 10d show competition binding assays with the subfragment u70.

Figure 11 and 11b show a location of binding sites in u50 and u70 by the methylation interference technique; Figure 11c provides a summary of these results.

Figure 12 shows an autoradiograph of binding complexes formed with u 70 in B cell and non B cell extracts.

Figure 13a is a restriction map of Kappa enhancer; Figure 13b shows a autoradiograph of binding assays with Kappa-Enhancer fragments; Figure 13c and 13d show an autoradiograph of competition assays with Kappa enhancer fragments.

Figure 14 shows location of Kappa-3 binding by methylation interference experiments.

Figure 15a shows binding analysis of Kappa-3 in various lymphoid and non-lymphoid cells; Figure 15b shows the binding analysis of Kappa-3 in cells at various stages of B-cell differentiation.

### Best Mode of Carrying our the Invention

The transcriptional regulatory factors described herein can be broadly classified as constitutive or tissue (lymphoid) specific. All factors are believed to play a role in transcription of Ig genes. Constitutive factors may have a role in regulating transcription of other genes as they are found in non-lymphoid cells.

The presence of constitutive factors rendered the detection of tissue specific factors more difficult. To facilitate detection, a sensitive DNA binding assay described below, was employed in all studies.

The characteristics of the transcriptional regulatory factors IgNF-A, E, IgNF-B and Kappa-3 are summarized in the chart below.

| Factor designation | Ig Regulatory Sequence | | | | | |
|---|---|---|---|---|---|---|
| | Promoter | | Enhancer | | Lymphoid | Nonlymphoid |
| | V_{H} | V_{K} | U_{E} | K_{E} | | |
| IgNF-A | + | + | + | - | + | + |
| E | - | - | + | + | + | + |
| IgNF-B | + | + | + | - | + | - |
| Kappa-3 | - | - | - | + | + | - |

As indicated in the chart, IgNF-A binds to Ig regulatory DNA elements in the region of mouse heavy and Kappa light chain gene promoters and also to mouse heavy chain gene enhancer. DNAase I footprint analysis indicates that the binding is mediated by the octomer sequence (ATTTGCAT) which occurs in mouse and human light chain gene promoters approximately 70 base pairs upstream from the site of initation and in heavy chain gene promoters at about the same position (in inverted sequence).

Deletion or disruption of the IgNF-A binding site in Ig promoters significantly reduces the level of accurately initiated transcripts in vivo. See, e.g., Bergman, Y. et al. PNAS USA 81 7041-7045 (1984); Mason, J.O. et al. Cell 41 479-487 (1985). As demonstrated below (See Exemplification), this also occurs in an in vitro transcription system. IgNF-A appears to be a positive transacting factor.

IgNF-A binding site appears to be a functional component of the B lymphocyte-specific Ig promoter. For example, sequences from this promoter containing the IgNF-A binding site specify accurate transcription in B cells but not in Hela cells. IgNF-A however, may not be restricted to B cells because a factor was detected in Hela cell extracts which generated complexes with similar mobilities and sequence specificty (as tested by competition analysis). Interestingly, the Ig octamer motif in the IgNF-A binding site has recently been shown to be present in the upstream region (about -225 bp) of vertebrate U1 and U2 snRNA genes. More importantly, this element dramatically stimulates (20 to 50 fold) transcription of U2 snRNA genes in Xenopus oocytes. Therefore, IgNF-A may be a constitutive activator protein that also functions in the high level expression of U1 and U2 snRNA genes in vertebrate cells.

The presence of an IgNF-A binding site in the mouse heavy chain enhancer suggests the additional involvement of IgNF-A in enhancer function. It is known that deletion of an 80 bp region of the enhancer containing the putative binding site reduces enhance activity approximately tenfold. The occupation of the binding site, in vivo, has been inferred from the fact that the G residue in the enhancer octamer protected from dimethyl sulfate modification only in cell of the B lineage. Furthermore, IgNF-A also binds in a sequence-specific manner to the SV40 enhancer (J. Weinberger, personal communication), which contains the Ig octamer motif, thereby strengthening the notion that the factor participates in enhancer function.

The E factor is a constitutive factor which binds to the Ig light and heavy chain enhancer.

Factor IgNF-B binds to the same regulatory elements as IgNF-A. Indeed, the binding site for IgNF-B appears to be the octomer motif. In contrast to IgNF-A, IgNF-B is lymphoid cell specific. It was found in nuclear extracts from pre B, mature B and myeloma cell lines and in nuclear extracts from some T cell lymphomas. IgNF-B was undetectable in nuclear extracts of several non-lymphoid cells.

Kappa-3 binds only to the Ig light chain enhancer. The factor is lymphoid cell specific. Kappa 3 is also lymphoid stage specific; it is expressed only by mature B-cells. In this case, it is a marker of B cell maturation (the factor can be used to type B cell lymphomas).

The transcriptional regulatory factors described above were identified in extracts of cellular nuclear protein by means of an improved gel electrophoresis DNA binding assay with enhanced sensitivity. This improved assay is a modification of an original assay based on the altered mobility of protein-DNA complexes during gel electrophoresis. The original assay has been extensively employed in equilibrium and kinetic analyses of purified prokaryotic gene regulatory proteins. See, e.g., Fried, M. and Crothers, D.M. Nucleic Acid Res. 9 6505-6525 (1981); Garner, M.M. and Revzin A. Nucleic Acids Res. 9 3047-3060 (1981). More recently it has been used to identify and isolate a protein that binds to satellite DNA from a nuclear extract of eukaryotic cells (monkey cells). See Strauss, R. and Varshavsky, A. Cell 37 889-901 (1984). In the latter study an excess of heterologous competitor DNA (E. coli) was included with the specific probe fragment to bind the more abundant, non-specific DNA binding proteins in the extract.

In the improved assay of this invention, the simple alternating copolymer, duplex poly(dI-dC)-poly(dI-dC) was used as the competitor DNA species. The use of this copolymer as competitor resulted in an enhancement of sensitivity for detection of nuclear protein-DNA complexes.

The assay is performed essentially as described by Strauss and Varshasky, supra, except for the addition of the poly(dI-dC)-poly(dI-dC). An extract of nuclear protein is prepared, for example, by the method of Dingnam, J.D. et al., Nucleic Acids Research 11:1475-1489 (1983). The extract is incubated with a radiolabled DNA probe that is to be tested for binding to nuclear protein. The incubation is carried out in the presence of the poly(dI-dC)-poly(dI-dC) competitor in a pysiological buffer. DNA protein complexes are resolved from free DNA probes by electrophoresis through a polyacrylamide gel in a low ionic strength buffer and visualized by autoradiography.

In a preferred embodiment, protein samples (about 10 ug protein) are incubated with approximately 10,000 cpm (about 0.5 ng) of an end-labeled ³²P doublestranded DNA probe fragment in the presence of about 0.8 - 4 ng poly(dI-dC).poly(dI-dC) (Pharmacia) in a final volume of about 25 ul. Incubations are carried out at 30° for 30-60 minutes in 10 mM Tris HCl (ph 7.5), 50 mM NaCl, 1 mM DTT, 1 mM EDTA. Protein-DNA complexes are resolved on low-ionicstrength polyacrylamide gels. Samples are layered onto low ionic-strength 4% polyacrylamide gels (0.15x16 cm; acrylamide:bisacrylamide weight ratio of 30:1). Gels are pre-electrophoresed for about 30 min at 11 V/cm buffer consisting of 6.7 mM TrisHCl, (ph 7.5), 3.3 mM NaOAc, and 1 mM EDTA. Buffer is recirculated between compartments. Gels are electrophoresed at the same voltage at room temperature, transferred to Whatman 3MM, dried and autoradiographed.

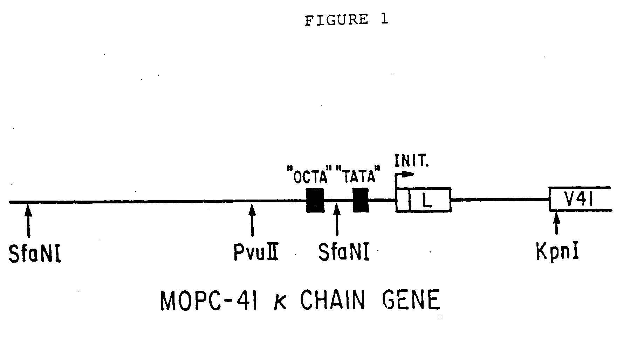

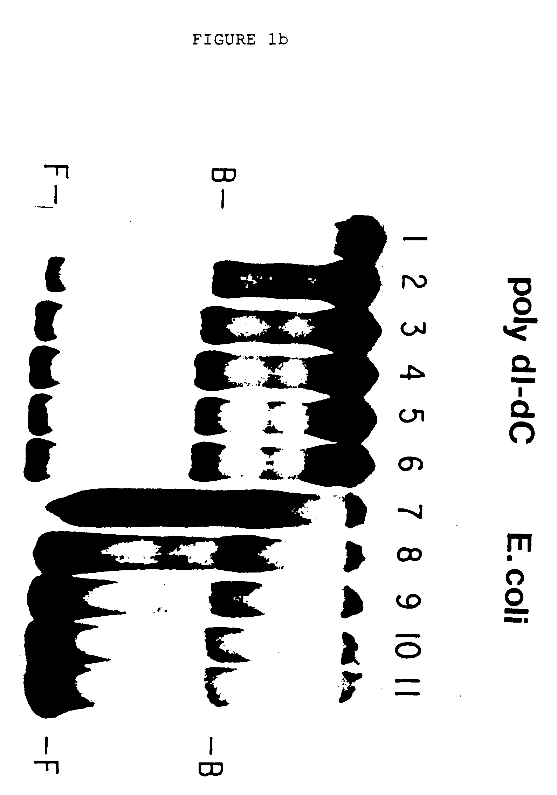

The enhanced sensitivity of the assay is illustrated by initial experiments leading to identification of the factor IgNF-A. A radiolabeled SfaNI-SfaNI DNA fragment derived from the upstream region of the MOPC 41 Kappa light chain gene (Figure 1a) was incubated with a nuclear extract of a human B cell line in the absence or in the presence of E. coli chromasomal DNA or poly(dI-dC)-poly(dI-dC). The resulting complexes were resolved from the free fragment by electrophoresis through a low ionic strength, non-denaturing polyacrylamide gel and visualized by autoradiography (Figure 1b). In the absence of competitor DNA all of the labeled fragment was retained at the top of the gel (lane 1) probably due to the binding of an excess of non sequence-specific proteins. With addition of increasing amounts of either poly(dI-dC)-poly(dI-dC) (lanes 2-6) or E. coli chromosomal DNA (lanes 7-11) as competitors, putative protein-DNA complexes which migrated slower than the free fragment were detected. The relative abundance of the major species of complex (B) as well as that of minor species was significantly greater in the presence of the alternating copolymer competitor DNA.

The use of a sensitive gel electrophoresis DNA binding assay in conjunction with the copolymer competitor poly(dI-dC).poly(dI-dC) facilitated the identification of the regulatory factors described herein. The simple alternating copolymer probably competes less effectively than heterologous sequences DNA for binding of a sequence-specific factor, thereby significantly increasing the sensitivity of the assay. The assay has general applicability for elucidation of mammalian gene regulatory proteins.

A further increase in sensitivity in this assay is obtained by the use of small DNA probes (about 100 bp or less) which minimize non-specific binding interactions in a crude extract.

Employing this assay, binding competition tests can be perfomed to analyze the sequence specificity of protein-DNA interactions. For this purpose, an unlabled DNA fragment to be examined for competitive binding to the protein factor can be added to the incubation mixture of protein extract and labeled DNA probe (along with the poly (dI-dC)-poly(dI-dC). The dissappearance of protein-DNA probe complex, or its diminishment, indicates that the unlabeled fragments competes for binding of the protein factor. In addition, relative binding affinity of the protein to a probe sequence can be assessed by examining the ability of a competitor to displace the protein at varying concentrations.

In conjunction with the competition assays, DNase I footprint analysis (See Galas, D. and Schmitz A. Nucl. Acids Res. 5 3157-3170 (1978) and Exemplification below) and methylation protection experiments (See, e.g., Ephrussi, A. et al. Science 227:134-140 (1985) can be used I to refine analysis of the binding domain of the protein factors.

The functional role of the factors in the regulation of the transciption can be assessed in several ways. A preferred technique for lymphoid cell factors entails the use of the in vitro transcription system developed from cells of lymphoid cell lineage. This system is described in detail in the Exemplification section below. The function of a factor can be indirectly assessed in this system by employing templates for transcription which are deleted of the binding domain of the factor. As has been noted above, deletion of the upstream sequence located between -44 and -79 bp from the cap site of the MOPC41 Kappa gene disrupts transcriptions in this system (This has also been noted in in vivo systems). The deleted region includes the IgNF-A binding site. This indicates that transcription of the template is dependent upon the factor - binding site and, inferentially upon the factor itself.

A direct way to assess the function of the factors is to show that transcription can be modulated by removal and replacement of the factor in the in vitro transcription system with an appropriate template. For example, the intact MOPC41 K promoter gene can be used as a template in the in vitro system described and transcription of this template can be assessed in the presence and absence of a factor (for instance, IgNF-B, a lymphoid specific factor). The factor can be removed from the lymphoid cell extract by chromatographic fractionation and then replaced. If the level of transcription is diminished in the absence and restored by replacement of the factor, a direct indication of the factors involvement in transcription is provided.

Lymphoid specific factors involved in positive regulation Ig gene transcription may provide a method for enhancement of immunoglobulin production in lymphoid cells. Lymphoid cells, such as monoclonal antibody producing hybridomas, can be transfected with multiple copies of the gene encoding a regulatory factory to induce greater production of Ig. For this purpose, the gene encoding a regulatory factory can be linked to a strong promoter. (The construct can be designed to incorporate a selectable marker as well). Multiple copies of the contruct can be inserted into the cell by transfection procedure such as electroporation. The cell can be transfected with multiple factors, including constitutive factors, where factors are determined to act in conjunction. By amplifying factor genes in this manner, overexpression of the regulatory factors can be induced in the transfected cell and consequently production of immumoglobulin enhanced.

The invention is further illustrated by the following exemplification.

### EXEMPLIFICATION

### I. Identification of Nuclear Factor IgNF-A

### A. METHODS

### 1. Gel electrophoresis DNA Binding assays with the SfaNI-SfaNI K promoter fragment.

The SfaNI fragment was subcloned into the SmaI site of pS64 (pSPIgV_{K}, provided by N. Speck). For binding analysis this fragment was excised from pSPIgV_{K} by digesting with Hind III and Eco RI. These latter sites flank the Sma I site in the polylinker of pSP64. After end-labeling with [-³²P]dATP and the large fragment of E. coli DNA polymerase I, the radiolabeled fragment was isolated by polyacrylamide gel electrophoresis. Binding reactions were performed and the reaction mixtures resolved by electrophoresis (Figure 1b). The ³²P-labeled fragment (about 0.5 ng, 10,000 cpm) was incubated with a nuclear extract of a human B lymphoma cell line (EW)(prepared by the method of Dignam, J.D. et al. Nucl. Acids Res. 11 1475-1489 (1983)) in the absence (lane 1) or presence of two different non-specific competitor DNAs (lanes 2-11). Binding reactions (25 ul) contained 10 mM Tris.HCl (pH 7.5), 50 nM NaCl, 1 nM DTT, 1 mM EDTA, 5% glycerol and 8 ug EW nuclear extract protein. Reactions 2-6 additionally contained 800, 1600, 2400, 3200 and 4000 ng, respectively, of poly(dI-dC)poly(dI-dC). Reactions 7-11 contained 300, 600, 900, 1200 and 1500 ng, respectively, of Hinf I digested E. coli chromosomal DNA. After a 30 min incubation at room temperature, the resulting complexes were resolved in a low ionic strength 4% polyacrylamide gel (acrylamide:bisacrylamide weight ratio of 30:1) containing 6.7 mM Tris.HCl (pH 7.5), 3.3 mM Na-acetate and 1 mM Na-EDTA. See Strauss, F. and Varshavsky, A. Cell 37 889-901 (1984). The gel was preelectrophoresed for 30 min at 11V/cm. Electrophoresis was carried out at the same voltage gradient for 90 min at room temperature with buffer recirculation. The gel was then dried and autoradiographed at -70°C with a screen. In Figure 1b, F and B indicate positions of free and bound fragments respectively.

Binding assays were performed as detailed above using 2400 ng poly(dI/dC).poly(dI-dC)) and the following DNA fragments: K SfaNI-SfaNI ( 0.5 ng, 10,000 cpm, lane 1), K PvuII-KpnI ( 0.5 ng, 10,000 cpm, lane 2), K PvuII-SfaNI ( 0.1 ng, 5000 cpm, lane 3) and pSP64 PvuII-EcoRI ( 0.2 ng, 5000 cpm, lane 4). The K PvuII-SfaNI fragment was derived from the plasmid PSPIgV_{K} by digesting with PvuII and EcoRI. The EcoRI site is in the polylinker and therefore this fragment contains 16 bp of polylinker sequence.

### 2. Binding competition analysis in nuclear extracts of human EW and HeLa cells.

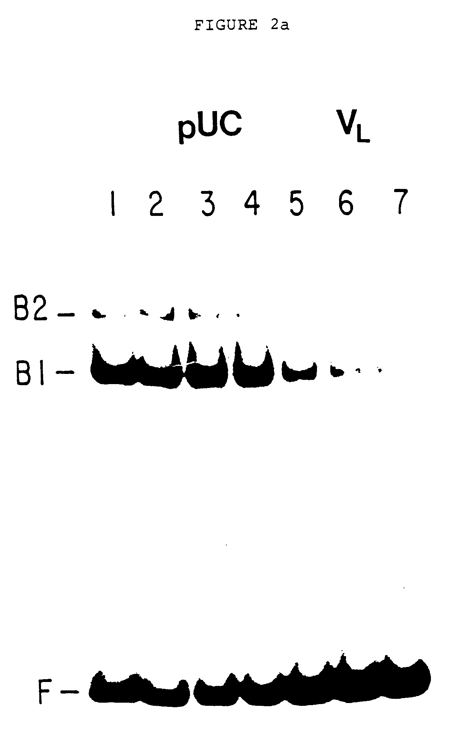

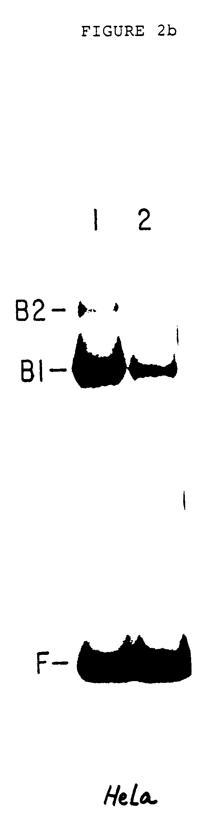

EW nuclear extract, Figure 2a. Binding assays were performed as detailed above using radiolabeled K PvuII-SfaNI fragment ( 0.1 ng, 5000 cpm) and 2400 ng poly(dI-dC).poly(dI-dC). Reactions 2-4 additionally contained 50, 100 and 200ng, respectively, of the bacterial plasmid pSP64 whereas 5-7 contained 50, 100 and 200 ng, respectively, of the recombinant plasmid pSPIgV_{K}. Assuming that a molecule of pSPIgV_{K} contains a single high affinity site whereas a molecule of pSP64 (3000 bp) contains 3000 non-specific sites, the apparent affinity ratio of the factor for these two types of sites is greater than 3000 x 200/50 = 1.2 x 10⁴ Hela nuclear extract, Figure 2b. Binding assays were performed as detailed above using radiolabeled K PvuII-SfaNI fragment ( about 0.1 ng, 5000 cpm), 2400 ng poly(dI-dC).poly(dI-dC) and 6 ug Hela nuclear extract protein (provided by P. Grabowski). Reactions 1 and 2 additionally contained 100 ng of pSP64 and pSPIgV_{K}, respectively.

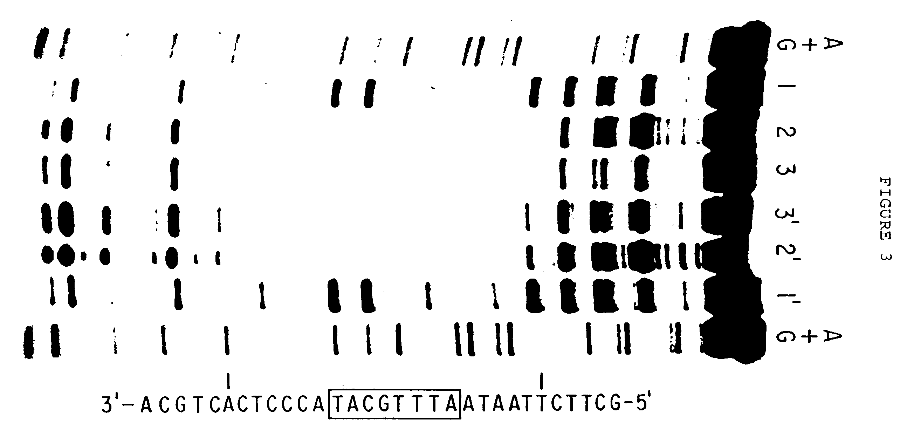

### 3. DNase footprinting of factor-DNA complexes. (Figure 3)

The B cell nuclear extract was applied to a heparin-sepharose column equilibrated with 10 nM Hepes pH 7.9, 20% glycerol, 1 mM DTT, 1 mM EDTA, 5 mM MgCl₂ and 0.1 M KCl. The K-promoter binding factor was eluted with a 0.25 M KCl step. Binding reactions with this fraction (30 ul) contained 2.5 mM MgCl₂, K PvuII-SfaNI ( 1 ng, 100,000 cpm) and 4800 ng poly(dI-dC).poly(dI-dC) in addition to components detailed above. The coding strand of the K promoter probe was 3' end-labeled (Eco RI site) with [ -³²P] dATP using the large fragment of E. coli DNA polymerase. Reaction 1 was digested with DNase I (5 ug/ml) for 2.5 min at room temperature in the absence of B cell nuclear protein. Reaction 2 was initially incubated with the heparin Sepharose fraction of the EW nuclear factor (14 ug protein) for 15 min at room temperature and then digested with DNase I as above. Each reaction was stopped with EDTA (5 mM) and the products separated by native polyacrylamide gel electrophoresis as detailed above. After autoradiography to visualize the various species, DNA was eluted from the free (reaction 1) and bound (B1 and B2, reaction 2) fragment bands by incubating gel slices in 0.5 M ammonium acetate (ph 7.5), 0.1% SDS and 1 mM EDTA with shaking at 37°C overnight. The supernatants were extracted sequentially with phenol-chloroformisoamylalcohol (25:24:1 v/v) and chloroform - isoamylalcohol (24:1 v/v) and precipitated with 2 volumes of ethanol in the presence of carrier tRNA. After a reprecipitation step the products were analyzed by separation in a 10% polyacrylamide gel (20:1) in the presence of 8M urea followed by autoradiography at -70°C with a screen. Lane 1 contains products of free fragment digestion from reaction 1. Lanes 2 and 3 contain digestion products eluted from bound bands B1 and B2, respectively, from reaction 1. Lanes 1', 2' and 3' corresponds to 1, 2 and 3, respectively, with the exception that the former set was digested with DNase 1 for 5 min. A-G chemical cleavage ladders of the K promoter probe were coelectrophoresed to map the binding domain. See Maxam, A. and Gilbert, W. Meth. Enzymol. 65, 499-525 (1980).

### 4. Binding of a common nuclear factor to three Ig transcriptional control elements.

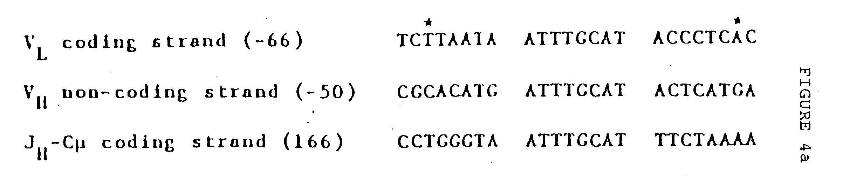

Nucleotide sequences of actual and putative binding sites, Figure 4a. The V_{L} binding site is defined by the DNase I protection assay (* indicates boundaries of the protected region). The V_{H} and J_{H}-C_{U} sequences are putative binding sites in the V_{17.2.25} promoter and the mouse heavy chain enhancer, respectively. Numbers in brackets indicate start coordinated of octamer motif. Binding competitions. Binding assays (10 ul) were performed as detailed above using 1600 ng poly(dI-dC)-poly(dI-dC) and the heparin Sepharose fraction of the EW nuclear factor (1.5 ug protein). V_{L} probe (about 0.1 ng, 5000 cpm) lanes 1-3, 10, 11. V_{H} probe ( 0.2 ng, 5000 cpm) lanes 4-6, 12-12. J_{H}-C_{H} probe ( 0.2 ng, 5000 cpm), lanes 7-9, 14, 15. Lanes 2, 5, 8 additionally contained 5 ng of a V_{L} promoter oligomer (36 bp, spanning positions -81 to -44 of the MOPC-41 V_{K} gene segment) whereas lanes 3, 6, 9 contained 50 ng of the same oligomer. Lanes 11, 13, 15 additionally contained 50 ng of a J_{H}-C_{H} oligomer (41 bp, spanning positions -1 to 40 of the heavy chain enhancer). Complementary single-stranded synthetic oligonucleotides were kindly made by Dr. Ronald Mertz, Genzentrum der Universitat Munchen and Dr. E. L. Winnacker, Institut fur Biochemie der Universitat Munchen. They were annealed prior to use as competition substrates in the binding assay.

### B. RESULTS

A radiolabeled SfaNI-SfaNI DNA fragment derived from the upstream region of the MOPC 4l kappa light chain gene (Fig. 1a) was incubated with a nuclear extract of a human B cell line in the absence or presence of two different competitor DNAs. The resulting complexes were resolved from the free fragment by electrophoresis through a low ionic strength, non-denaturing polyacrylamide gel and visualized by autoradiography (Figure 1b). In the absence of competitor DNA all of the labeled fragment was retained at the top of the gel (lane 1) probably due to the binding of an excess of non sequence-specific proteins. With addition of increasing amounts of either poly(dI-dC)-poly(dI-dC) (lanes 2-6) or E. coli chromosomal DNA (lanes 7-11) as competitors, putative protein-DNA complexes which migrated slower than the free fragment were detected. The relative abundance of the major species of complex (B) as well as that of minor species was significantly greater in the presence of the alternating copolymer competitor DNA. Because substitution of the simple copolymer considerably increased the sensitivity of the assay, it was used in all subsequent binding analyses.

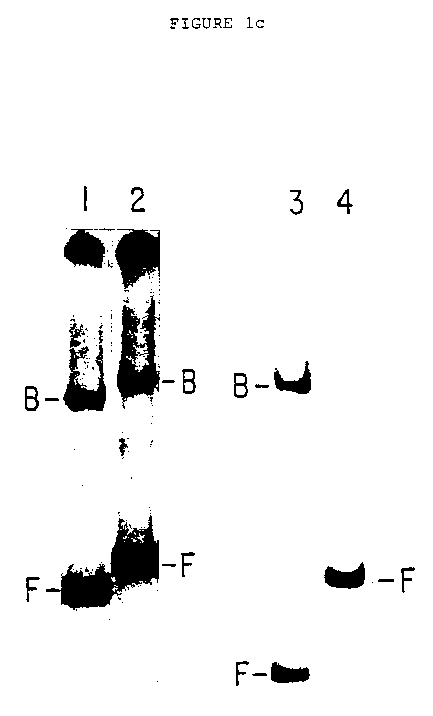

To test the sequence-specificity of the major species detected in the binding assay, a set of mutually overlapping Kappa promoter fragments (See Figure 1a, SfaNI-SfaNI, PvuII-KpnI and PvuII-SfaNI) and a similar length fragment derived from the bacterial plasmid SP64 (EcoRI-PvuII) were individually assayed (Figure 1c). Whereas the bacterial DNA fragment showed no appreciable binding (lane 4) all of the Kappa promoter fragments yielded major discrete complexes of similar mobilities (lanes 1-3). The mobilities of a series of complexes formed with different length fragment probes (75-300 bp) are approximately the same (data not shown). These data therefore suggested the binding of a specific nuclear factor within the region of overlap of the Kappa promoter fragments. This region includes the conserved octameric sequence but not the TATA element. Note that with the smallest 75 bp Kappa promoter fragment (lane 3) no appreciable label was retained at the top of the gel. Thus, as has been noted recently, the use of small probe fragments further enhance the sensitivity of detection of specific protein-DNA complexes.

The sensitivity gained by use of both the copolymer and a small fragment probe permitted the detection of two complexes, B1 and B2 (Figure 2a, lane 1). The major species B1 corresponds to complex B in the earlier figure. The relative affinity of the factor(s) for Kappa promoter DNA was estimated by a competition assay (Figure 2a). Whereas a control plasmid (pSP64), when added in the above incubation, failed to compete for binding in the concentration range tested (lanes 2-4), the recombinant plasmid (pSPIgV_{K}) effectively reduced the formation of both species B1 and B2 in the same range (lanes 5-7). The latter plasmid was constructed by insertion of the upstream region of the Kappa promoter into pSP64. Assuming that the pSPIgV_{K} plasmid contains a single high affinity binding site these results suggest that the nuclear factor(s) responsible for B1 and B2 has at least a 10⁴-fold higher affinity for its cognate sequence than for heterologous plasmid DNA (see Methods section 2 above).

To determine if the factor(s) responsible for formation of B1 and B2 was specific to B lymphocytes, a nuclear extract derived from human HeLa cells was assayed for binding to the Kappa promoter probe (Figure 2b). Both species B1 and B2 were generated at similar levels, as that observed with B cell extracts, by the HeLa extract (lane 1). Furthermore, both were specifically competed by the pSPIgV_{K} plasmid (lane 2). Thus HeLa cells also contain a factor(s) which binds specifically to the Kappa promoter upstream region.

DNase I footprint analysis was used to delineate at a higher resolution the binding domain(s) of factor(s) present in complexes B1 and B2. To facilitate these studies, the binding factor(s) from B cells was partially purified by chromatography of nuclear extract protein on a heparin sepharose column. Most of the binding activity eluted in a 0.25 M KC1 step fraction, giving a purification of approximately 5-fold (data not shown; see legend to Fig. 3). For footprint analysis, DNase I was added for a partial digestion after incubation of the partially purified factor(s) with the Kappa promoter probe B1 and B2 species were then resolved from free fragment by polyacrylamide gel electrophoresis. Bound DNA was eluted from both B1 and B2 bands and examined by denaturing polyacrylamide gel electrophoresis (Fig. 3, lanes 2, 3 and 2', 3'). DNase I digests of the Kappa promoter probe in the absence of B cell protein (lanes 1 and 1') and A+C chemical cleavage ladders were coelectrophoresed to map the binding domain. Factor(s) in the B1 complexes (lanes 2 and 2') appeared to protect a 19 nucleotide region on the coding strand. The 5' and 3' boundaries of the protected region map to positions -72 and -52, respectively, from the site of transcriptional initiation. Queen, C. and Baltimore, D. Cell 32 741-740 (1983). The region of DNase I protection was centered about the conserved octanucleotide sequence ATTTGCAT suggesting its importance in the recognition of the Ig promoter by the nuclear factor. B2 complexes showed a virtually identical DNase I protection pattern as B1 complexes and therefore do not appear to involve additional DNA contacts (lanes 3 and 3'). The simplest interpretation of this observation is that the B2 complex is generated by dimerization through protein-protein interactions of the factor responsible for the B1 complex. Alternatively, the B2 complex could be formed either by the binding of another protein to the factor responsible for the B1 complex or by recognition of the same set of sequences by a distinct DNA binding protein.

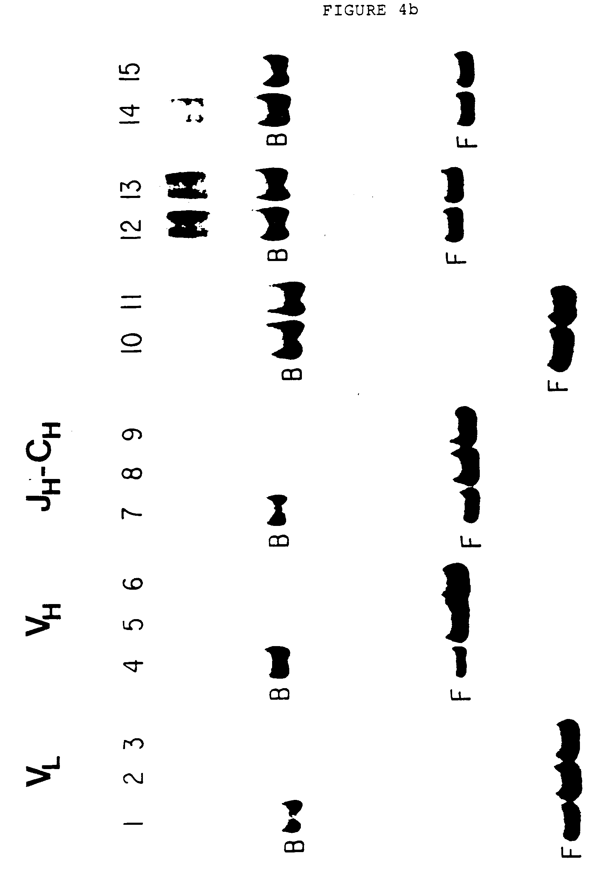

Because the octamer sequence motif is present in both light and heavy chain gene promoters as well as in the enhancer elements of both mouse and human heavy chain genes, assays were performed for factor binding to fragments from a mouse heavy chain promoter (V_{H}) and the mouse heavy chain enhancer. The V_{H} promoter fragment was derived from the 5' region of the V_{17.2.25} gene and included nucleotides between positions -154 and +57 relative to the transcriptional start site. Grosscheal, R. and Baltimore, D. Cell 41 885-897 (1985). In this promoter the conserved octanucleotide spans positions -57 to -50 (Figure 4a). The heavy chain enhancer fragment was derived from the germline J_{H} C_{U} region and spanned positions 81 to 251 within a 313 bp region implicated in enhancer function. Banergi, T. et al. Cell 33 729-740 (1983). The conserved octanucleotide is positioned between coordinates 166 to 173 in the above fragment (Fig. 4a). The B-cell heparin sepharose fraction (purified on the basis of binding to the Kappa promoter sequence, Fig. 4b, lane 1) evidenced binding to both the V_{H} promoter fragment (lane 4) and to the enhancer fragment (lane 7). The mobilities of the complexes formed with the three fragments were very similar consistent with the binding of a common factor. Furthermore, binding to these fragments was specifically competed by a synthetic duplex 40-mer that spanned the octanucleotide motif of the MOPC 41 Kappa light chain gene promoter (lanes 1-9). An oligomer of equivalent size containing a sequence from a region of the mouse heavy chain enhancer lacking the octanucleotide motif (Fig. 4b) failed to compete for binding in the same concentration range (lanes 10-15). This competition analysis further strengthens the suggestion that a common nuclear factor (IgNF-A) binds to all three I_{g} transcriptional elements. As has been mentioned previously, these three transcriptional elements share an identical sequence motif ATTTGCAT (Fig. 4a). Thus, the binding of a common nuclear factor is almost certainly mediated by this motif.

### II. Dependency of in vitro transcription of Ig genes on an upstream sequence

### A. METHODS

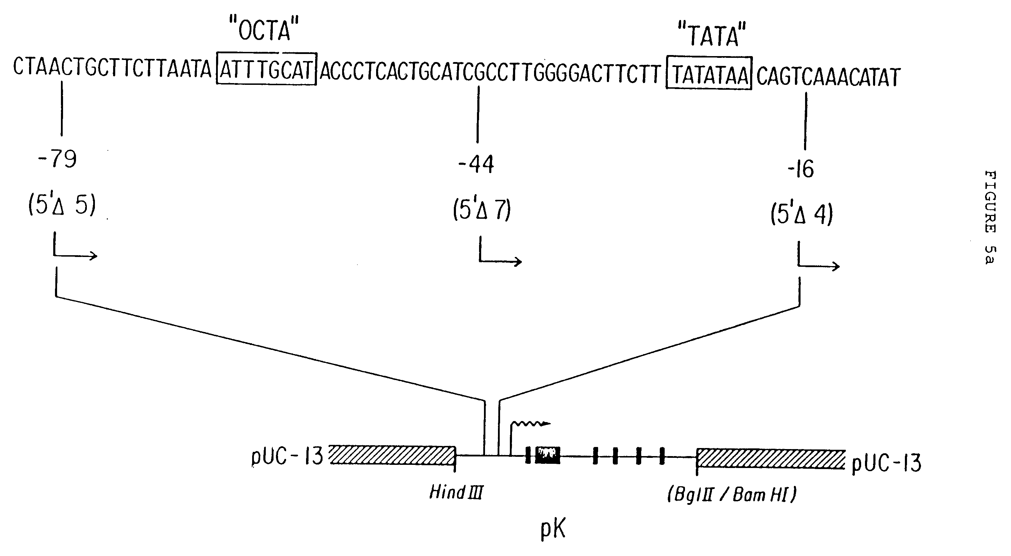

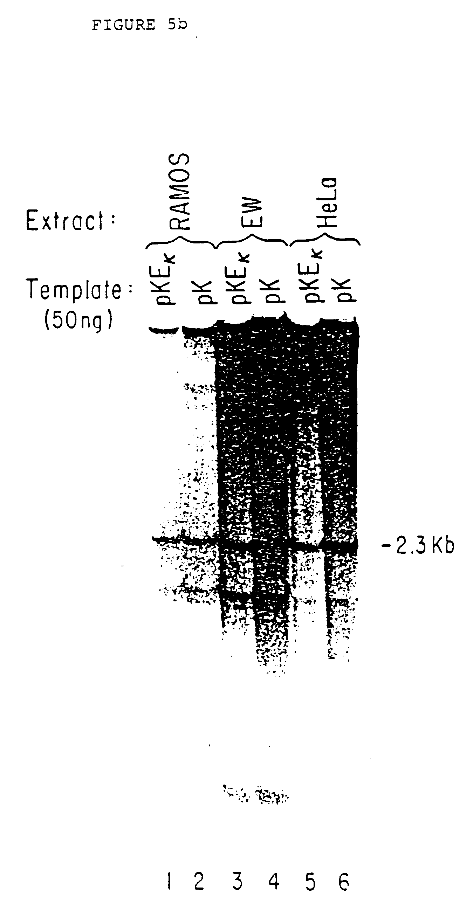

Figure 5a: Templates. The deletions 5' 5 and 5' 7 have been described before. See Bergman Y. et al PNAS USA 81:7041 (1981). The highly conserved octanucleotide sequence which is found upstream of all sequenced heavy and light chain variable region genes is boxed (labelled "OCTA"). It is located approximately 30 base pairs upstream from the "TATA" box. The plasmids pK and p K were constructed by converting the 5'-ends of 5' 5 and 5' 7 into a Hind III site by means of synthetic linkers followed by cloning the fragment up to the Bgl II site in the J_{K}-C_{K} major intron into Hind III, Bam HI digested pUC-13. pKE_{K} and pKE_{K} represent plasmids containing either the kappa enhancer of the heavy chain enhaner cloned into the unique Hind III site of pK. The segments used as the enhancers are an 800 bp Hind III-Mbo II fragment from the J_{K}-C_{K} intron (Max, E.E. et al. (1981) J. Biol. Chem. 2565116) and a 700 bp Xba I-Eco RI fragment from the J_{H}-Cₖ intron. Gillies, S.D. et al. Cell 33:717 (1983); Banjerji, J. et al. Cell 33:729 (1983). Figure 5b; transcription in whole cell extracts made from the human B lymphoma cell lines RAMOS (lanes 1,2) and EW (lanes 3,4); transcription in a HeLa whole cell extract (from A Fire (lanes 5,6)). The expected 2.3 kb run off transcript is indicated.

The cell lines RAMOS and EW were grown in RPMI medium containing 10% inactivated fetal calf serum to a density of 5-8x10⁵ cells per ml. Whole cell extracts were generated according to the procedure of Manley et al., PNAS USA 77:3855 (1980), and had a final protein concentration of approximately 18 mg/ml. Run off transcription reactions were carried out at 30° for 60' in a reaction volume of 20 ul. A typical reaction mix contained 9 ul (160 ug) of whole cell extract, 50 uM each of ATP, CTP and GTp, .5 uM UTP, 10 u Ci of a-³²P UTP (NEG 007X, 7600 Ci/mM) 5 mM creatine phosphate, 0.3 mg/ml creatine phosphokinase (Sigma), 12 mM Hepes 7.9, 12% glycerol, 60 mM KC1, 5 mM MG⁺⁺, 1 mM EDTA, 0.6 mM DTT, linearized template (about 50 ng) and poly (dI-dC)-poly(dI-dc) as a non-specific carrier (about 400 ng). The reactions were terminated by adding 200 ul of stop buffer (7M urea, 100 mM LiCl, 0.5% SDS, 10 mM EDTA, 250 ug/ml tRNA, 10 mM Tris (pH 7.9), followed by two extractions with phenol: chloroform: isoamyl alcohol (1:1:0.05), one with chloroform and precipitation with ethanol. The RNA's were treated with glyoxal and analyzed by electrophoresis through a 1.4% agarose gel in 10 mM sodium phosphate (pH 6.8), 1 mM EDTA. See Manley et al. supra. The gel was then dried for autoradiography with an intensifying screen at -70°C.

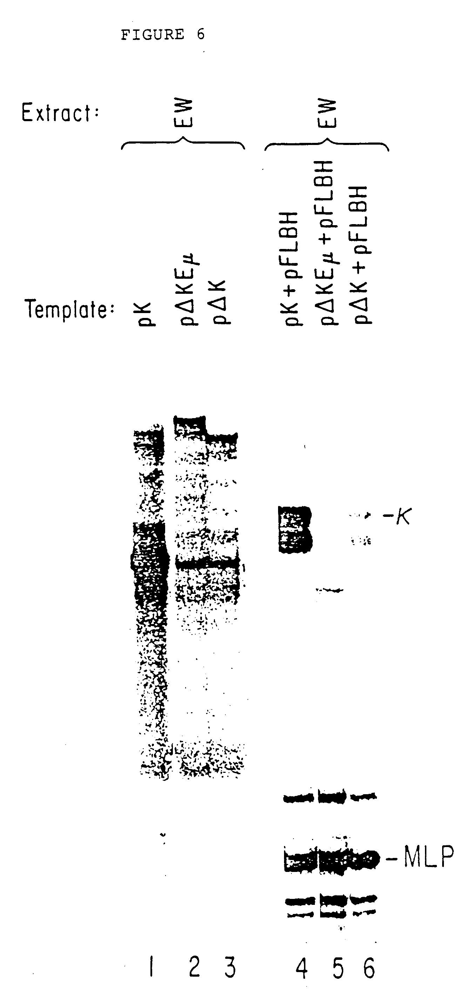

Figure 6: Effect of the upstream deletion 5' 7 on in vitro transcription in B cell extracts utilizing a pre-incubation pulse chase protocol. Run off transcripts obtained utilizing templates containing either the wild type promoter (lane 1) or the truncated Kappa promoter (lanes 2,3). Lanes 4-6: In vitro transcription using closed circular templates containing the wild type promoter (lane 4) or the truncated Kappa promoter (lanes 5-6). In these reactions 50 ng of a closed circular template containing the adenovirus major late promoter (MLP) was included as an internal control. The transcripts specific to the kappa template or the adenovirus template are indicated as Kappa and MLP, respectively. For a template containing the major late promoter the plasmid pFLBH was used. The plasmid contains sequences from 14.7 to 17.0 map units of adenovirus inserted between the Bam HI and Hind III sites of pBR322 and was the kind gift of A. Fire and M. Samuels.

Either the linearized or the supercoiled template (50 ng) was incubated in a volume of 20 ul with 9 ul (about 150 ugm) of EW extract, 6% (wt/vol) polyethylene glycerol 20,000 and all other components described for Fig. 5b except the nucleotides for a period of 60 minutes at 30°C. Transcription was initiated by the addition of nucleotides and radioactive UTP to the following final concentrations: 60 uM each of ATP, CTP and GTP and 1 uM UTP and 10 uCi -³²P UTP (NEG 007X, 600 Ci/mM). The initiating pulse was maintained for 10' at 30° followed by a 10' chase with a vast excess of non-radioactive nucleotides. Final concentrations during the chase were as follows: 330 uM ATP, CTP, GTP and 1 mM UTP. The reactions were quenched, worked up and the run off transcripts analyzed as described above. Mapping of the initiation site of the transcript was conducted as follows: Transcripts generated from closed circular templates were taken up in 20 ul of HE (50 mM Hepes, pH 0.7, 1 mM EDTA) and 10 ul was used for hybridization selection. A hybridization template complementary to the Kappa RNA was constructed by cloning the Pva II-Sau 3A fragment which contains the cap site of the MOPC41 gene (Queen and Baltimore, Cell 33:741 (1983)) into the M13 phage MP9. Single stranded phage DNA was prepared and purified by density gradient centrifugation through cesium chloride. MLP specific transcripts were detected using the M13 clone XH11 provided by A. Fire and M. Samuels. Hybridizations were done in a final volume of 15 ul in the presence of 750 mM NaCl and 100-200 ug of single stranded complementary DNA at 50°C for 2 hrs. The reactions were then diluted with 200 ul of cold quench solution (0.2 M NaCl, 10 mM Hepes pH 7.5, 1 mM EDTA) and 2 U of ribonuclease T1 added. Digestion of single stranded RNA was allowed to proceed for 30' at 30°C after which the reactions were extracted once with phenol chloroform isoamyl alcohol (1:1:.05) and precipitated with carrier tRNA. The pellet was washed once with cold 70% ETOH, dried and resuspended in 80% v/v formamide, 50 mM Tris borate, pH 8.3 and 1 mM EDTA. The RNA was denatured at 95°C for 3 min and then electrophoresed through a 6% polyacrylamide 8.3 M urea sequencing gel. The upper of 2 bands (Kappa) derived form the immunoglobulin promoter represent the correct start for Kappa transcription. The lower band is seen at variable intensities and probably does not represent a different cap site, as explained below.

### B. RESULTS

Whole cell extracts were made from two human Burkitt lymphoma lines, EW and RAMOS, by the procedure of Manley et al. supra. The templates used for in vitro transcription reactions are diagrammed in Fig. 5a. The template representing the wild type gene (pK) was derived from the MOPC41 Kappa gene and contained sequences from approximately 100 bp upstream from the transcription initiation site (end point 5' 5, Fig. 5a) to the Bgl II site in the major J_{K}-C_{K} intron Max, E.E. J. Biol. Chem. 256:5116. This fragment retains the complete variable region which is rearranged to J_{K}l, but not the Kappa enhancer which is further downstream of the Bgl II site. See, e.g., Queen and Stafford, Mol. Cell. Biol. 4:1042 (1984). This short 5' flank has been shown to be sufficient for accurate initiation and high level of transcription in a transient transfection assay. Bergman, Y. et al. PNAS USA 81:7041 (1984). Deletion analysis of the Kappa promoter showed previously that important regulatory sequences are present between nucleotides -79 and -44 because deletion 5' 7 completely abolished transcriptional competence of the gene while deletion 5' 5 had no effect. See Bergman et al. supra. The template representing an inactive promoter mutant (p K) was constructed by engineering a Hind III site into the 5' -end of 5' 7 and cloning the segment of the gene up to the Bgl II site into pUC13 cut with Hind III and Bam HI.

To examine transcriptional activity in B cell extracts, a linear template truncated at the Sac I site in the polylinker was used and transcripts ending at this site (run off transcripts) were examined by electrophoretic separation. A run off transcript of 2.3 kb was evident when RAMOS, EW or HeLa cell extracts were used (Fig. 5b, lanes 2, 4 and 6). When a Kappa chain enhancer sequence was added to the construct, no effect was evident implying that transcription in these extracts is enhancer independent (Fig. 5b, lanes 1, 3 and 5). (In EW and HeLa, the enhancer appears to cause a slight increase in the background radioactivity but not in the 2.3 kb band.) The band at 2.3 kb could be abolished by not adding the template or by transcribing in the presence of 0.5 ugm/ml amanitin. Thus it represents a template-specific, RNA polymerase II transcript. The band just below 2.3 kb is not decreased by -amanitin and presumably reflects end-labeling of endogenous 185 rRNA.

To assess whether initiation of transcription occurred at the natural cap site, a second assay was used. See Hansen, U. and P.A. Sharp (1983) EMBOJ 2:2293. For this assay, the uniformly labeled RNA was hybridized to a single stranded DNA probe spanning the transcription initiation site (generated by cloning the Pvu II-Sau 3A fragment of the Kappa gene into phage M13). The resulting complex was digested with ribonuclease T1 and the ribonuclease-resistant RNA fragments were analyzed by electrophoresis through a 6% polyacrylamide gel with 8.3 M urea. Analysis of in vitro synthesized RNA by this method is shown in Fig. 6 (lane4). The upper band (labeled Kappa) represents the correct cap site. The band just below it was seen at variable intensities and probably does not represent a different cap site but rather arises from cleavage with ribonuclease T1 at the next G residue from the 3'-end of the protected region. (Examination of the sequence near the Sau 3A1 site shows that the second set of G residues on the RNA lies 19 bp upstream from the end of the region of homology with the single stranded DNA probe). Thus the extracts generated from B cells were capable of correctly initiating and transcribing the immunoglobulin promoter in vitro with approximately the same efficiency as a HeLa cell extract.

To analyze the effect of 5' flanking sequences in vitro, we examined the transcription of the deleted gene, p K. Because many reulatory effects act upon the rate of initiation of transcription, we chose to use a pre-incubation, pulse-chase protocol which measures the initiation rates. See Fire, A. et al. J. Biol. Chem. 259:2509 (1984). The template DNA was first pre-incubated with the extract to form a pre-initiation complex. Transcription was then initiated by the addition of nucleotides and radiolabeled UTP. The initiated transcripts were completed during a chase period with unlabelled nucleotides and analyzed by electrophoretic separation. Incorporated radioactivity in this assay is proportional to the number of correct initiations occurring during the pulse.

In Fig. 6, comparison of lanes 2 and 3 with lane 1 shows that the template pK, which contains about 100 bp upstream of the initiation site, initiated approximately 10-fold more efficiently than did the deletion mutant, pK. Again, the presence of the heavy chain enhancer placed at -44 bp to the truncated promoter did not alter the level of transcription. When closed circular templates were used, a similar effect of the promoter truncation was observed (Fig. 6, lanes 4-6). In these reactions a template containing the major late promoter of adenovirus was included as an internal control; the expected protected RNA fragment of 180 bp is labeled MLP. Comparison of lanes 5 and 6 with lane 4 shows that there was a 10-fold decrease in the efficiency of transcription from the mutant promoter, whereas the transcript of the major late promoter remained constant. The reason for the apparent decrease in the amount of transcription from the supercoiled template containing the heavy chain enhancer has not been further addressed. It is evident, however, that the dependence of transcription on an upstream sequence between -44 and -79 is observed whether the effect described above was specific to B cell extracts, the same templates were transcribed in the heterologous HeLa whole cell extract. A 4- to 5-fold decrease in transcription was seen with the deleted template when compared with the wild type template (data not shown). Thus, the effect of the deletion is at best, only modestly tissue-specific.

We have reported here the development of transcriptionally competent whole cell and nuclear extracts from two independent human B cell lymphomas. In such extracts, transcription from the promoter of the MOPC41 Kappa gene was correctly initiated and a promoter deletion significantly reduced the level of initiated RNA. In vitro, the effect of the deletion used here is several hundred-fold when analyzed by a transient transfection assay, however the effect we observe in vitro is only about 10- to 15-fold. Although there are now several examples of upstream sequence requirements for in vitro transcriptions, See, e.g., Groschedl R. and Birsteil M.L. (1982) PNAS USA 79:297; Hen, R. et al. (1982) PNAS USA 79:7132, the effects have been smaller than the corresponding one in vitro. This is possibly due to the dominance of the TATA box and associated factors in determining the level of transcription in vitro Miyamoto, N.G. et al., Nucl. Acids Res. 12:8779 (1984).

### III. Discovery and Characterization of IGNF-B

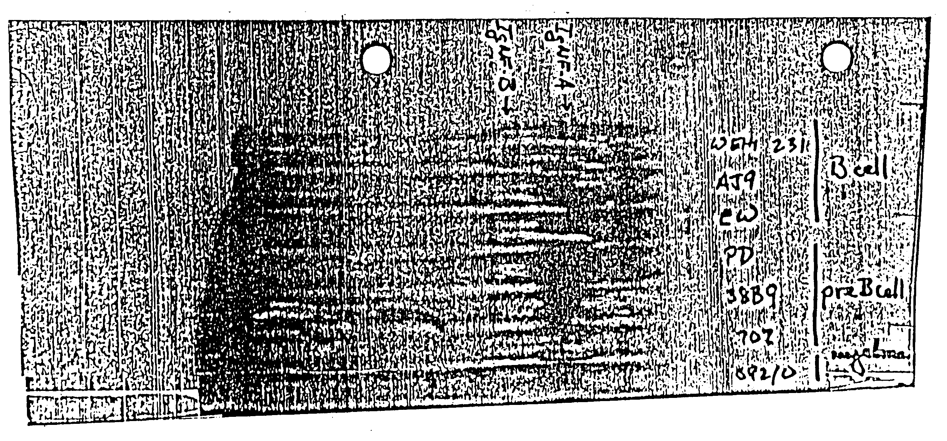

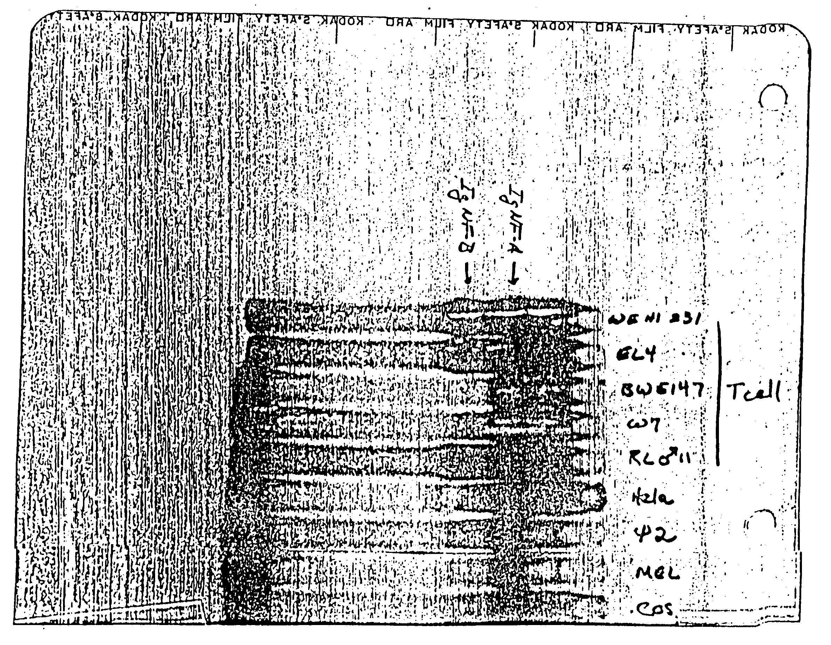

The mobility shift gel electrophoresis assay was used to screen nuclear extracts from a variety of cell lines for octamer binding proteins. The band corresponding to IgNF-A was found in all extracts but a second band with distinct mobility from IgNF-A was found only in nuclear extracts from lymphoid cells. This lymphoid-specific octamer binding protein, termed IgNF-B, was found in nuclear extracts from all pre-B, mature B and myeloma cell lines tested and in nuclear extracts from some T cell lymphomas (see Figure 7 and 8). IgNF-B was not detected in nuclear extracts from the non-lymphoid cell lines, Hela, 2, Cos and Mel (see Figure 8). IgNF-B was shown to be specific for the same octamer sequence as IgNF-A by competition experiments n which the IgNF-B band was selectively competed by unlabelled DNA fragments sharing only the octamer sequence and not by DNA fragments lacking the octamer sequence.

The octamer sequence is found at approximately position -70 upstream of the transcription start site of all immunoglobulin (Ig) variable genes which is in the region that has been shown to control the lymphoid specificity of the Ig promoter. Thus IgNF-B may bind to the upstream octamer sequence in lymphoid cells and activate transcription.

### IV. Factors Binding to u-Enhancer: E Factor

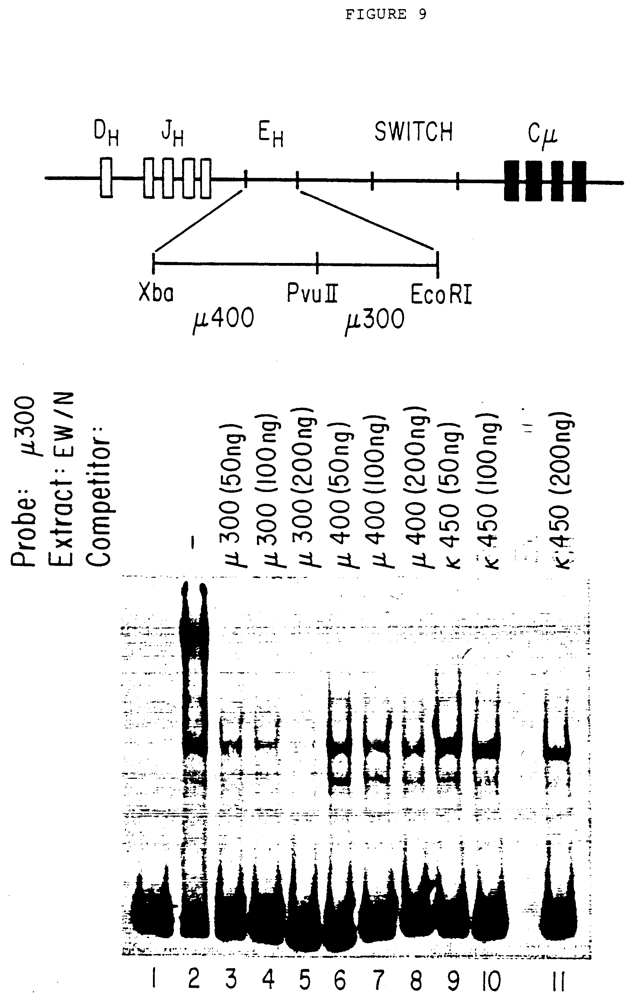

The fully functional u enhancer has been localized to a 700 bp XbaI EcoRI fragment from the major intron between J_{H} and C_{U}. This fragment can be further subdivided by cleaving at the PstI site to generate a 400 bp Xbal-PstI fragment (u400) and a 300 bp PstI-EcoRI fragment (u300). It has been shown by transient transfections that 30-50% of the tissue specific enhancer activity is retained in u300, whereas there is no detectable activity of u400. The gel binding assay was employed to investigate what protein factors may interact with the u-enhancer. Briefly, end-labelled DNA fragments were incubated with nuclear extracts made from tissue culture cells. After 20 min at room temperature the mixture was loaded on a low ionic strength polyacrylamide gel and electrophoresis carried out at 120V for 2 hrs. The gels were then dried for autoradiography. When the functional 300 bp (u300) enhancer fragment was used in such an assay a DNA-protein complex was observed in extracts derived from the human B lymphoma cell line EW (Figure 9b, lane 2). To show that this new band represented a specific complex binding reactions were carried out in the presence of varying amounts of non-radioactive competitor fragments (Figure 9b, lanes 3-11). It is easily seen that when u300 is added as the competitor fragment, the complex band is completely lost. In contrast, the adjacent u400 fragment (lanes 6,7,8) or a 450 bp fragment containing the K light chain enhancer (lanes 9,10,11), cause only a minor effect even at the highest concentrations used. It is interesting to note that there appears to be a slight increase in the amount of specific complex in the presence of the K enhancer fragment (compare lanes 9 and 2). As demonstrated below, both the u and the K enhancers interact with at least one common protein and this is not factor being detected by binding the u300 fragment. The increase in the specific complex in the presence of the K enhancer is probably due to the removal of factors common to both the enhancers from the reaction mix, thus leaving more of the labelled fragment available to bind to the u specific factor being detected by it.

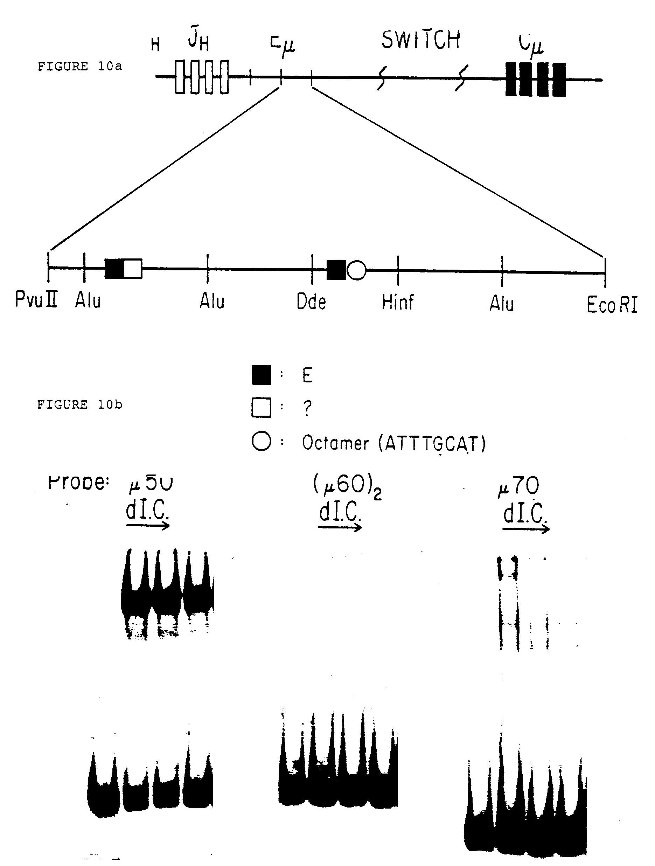

In order to be able to detect binding sites for less abundant proteins and also to more precisely define the complex detected with u300, this fragment was further dissected. Each of the smaller fragments generated were analyzed for their ability to serve as binding sites for nuclear proteins. Figure 10a shows a partial restriction map of the relevant region of the u enhancer. u300 was digested with AluI, HinfI and DdeI to generate a number of 50-70 bp fragments labelled u50, u(60)₂ (a mixture of AluI-DdeI and HinfI to AluI) and u70 (AluI-AluI). Binding reactions were carried out with each of these fragments with nuclear extracts of EW lymphoma cells in the presence of increasing amounts of the non-specific competitor poly (dI-dC)-poly(dI-dC). The results are shown in Figure 10b.

Fragment u50 forms a major complex band (lanes 2,3,4) that is barely decreased even in the presence of 4 ug of poly(dI-dC)-poly(dI-dC) (lane 4). The mixture of the two 60 bp fragments does not give rise to a discrete complex band (lanes 6,7,8). Finally the u70 fragment gave 2 faint, but discrete, nucleoprotein complex bands (lanes 10,11,12) of which the lower one is again barely affected by 3 ugm of non-specific carrier poly(dI.C).(dI.C) (lane 12).

Specificity of the complexes observed were shown by competition experiments using a variety of DNA fragments, Figure 10c. Thus, the complex generated with u50 is specifically competed away in the presence of u300 (of which u50 is a part), or a K promoter fragment, but not by corresponding amounts of u400 or a K enhancer fragment, consistent with the complex being generated by the interaction of the previously described factor IgNF-A with its cognate sequence. (This factor recognizes a conserved octanucleotide, ATTTGCAT, found in the promoters of all sequenced immunoglobulin genes and within this subfragment of the heavy chain enhancer.

The complex observed with u70 was specifically competed away by itself (lane 9) and to some extent with the K enhancer (lanes 5,6 Figure 10c) but not at all by either the Moloney murine leukemia virus enhancer (lanes 3,4) or by u400 (lanes 7,8, Figure 10d). Further competition analysis showed that this complex could not be competed away by either (u60)₂ (Figure 2D, lanes 7,8), u50 (Figure 2d, lanes 5,6) or u170 (central AluI-AluI fragment) (Figure 10d, lanes 11,12). The binding we have observed is therefore specific to this small fragment and was detected only upon further dissecting u300 which separated the major observable interaction of IgNF-A with the enhancer sequence to another fragment (u50).

Ephrussi et al. and Church et al. have used methylation protection experiments to define a set of G residues within the heavy chain enhancer that are specifically resistant to methylation by DMS in B cells. This result lead to the proposal that tissue-specific DNA binding proteins were responsible for this decreased accessibility of the reagent to DNA. The protection was observed 4 clusters, the DNA sequences of which were sufficiently homologous to derive a consensus sequence for the binding site of a putative factor. All four postulated binding sites (E1-E4) are found within the 700 bp fragment; however u300 retains only 2 complete binding domains (E3 and E4) for this factor and the octamer (0) sequence. The Alu-Alu fragment that shows that specific nucleoprotein complex described above contains the complete E3 domain and the factor we detect in vitro presumably is the same as that detected in vivo. Thus, it was unexpected that the HinfI-Dde fragment (u50) containing E4 and 0 should not compete for binding to u70 (Figure 10d, lanes 5,6).

In case this was due to the fragment predominantly binding IgNF-A at the octamer site and thus making it unavailable as a competitor for u70, binding reactions and competition assay were done for a fraction generated by chromatography of the crude extract over a heparin-sepharose column, that contained u70 binding activity and was significantly depleted of IgNF-A. When u50 or u170 was end-labeled and incubated with the column fraction, no specific nucleoprotein complexes were seen upon electrophoretic analysis. Even in this fraction, u50 and u170 failed to compete successfully for the interaction between u70 and its binding protein (data not shown), thus implying strongly that the binding site defined as E4 perhaps does not bind the same factor that binds at E3. Similarly, the E1 domain (isolated as a Hinf-PstI fragment) does not compete as effectively as u70 itself for the binding of the factor to u70.

To determined the location of the binding sites within individual fragments (u70 and u50), the technique of methylation interference was employed. End-labelled DNA fragments were partially methylated on guanines and adenines using dimethyl sulfate (DMS). Methylated DNA was then used for binding reactions with crude extracts and the complex was resolved from the free fragment by electrophoresis. Both complex and free fragment bands were then excised from the gel, and the DNA was recovered by electroelution. Piperidine cleavage of the recovered fragments was followed by electrophoresis through 12% polyacylamide urea sequencing gels. In principle, if any of methyl groups introduced by reaction with DMS interfere with the binding of a specific protein then that molecule of DNA will be selectively missing in the complex formed and subsequently the corresponding ladder. The method therefore allows identification of G residues making intimate contacts with the protein. [We have found that the use of DNaseI footprinting via the gel binding assay to be complicated in the case of some of these less abundant factors because of the short half lives of the complexes themselves. Thus if a binding incubation is followed by partial DNaseI digestion, it is possible that in the course of time required to load the sample and have the complex enter the gel, DNA fragments that were in complex form may exchange with the larger amounts of free fragment in the binding reaction. Thus not leading to any distinction in the DNase patterns seen with wither the complex or the free DNA (e.g. the half life of the nucleoprotein complex in u70 is less than 1 minute, Sen and Baltimore, unpublished observation)].

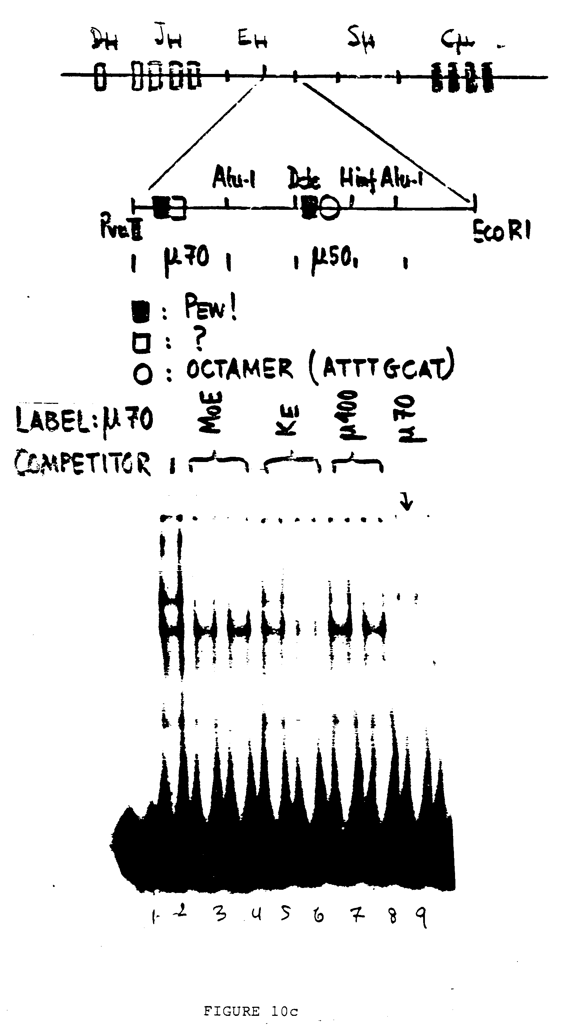

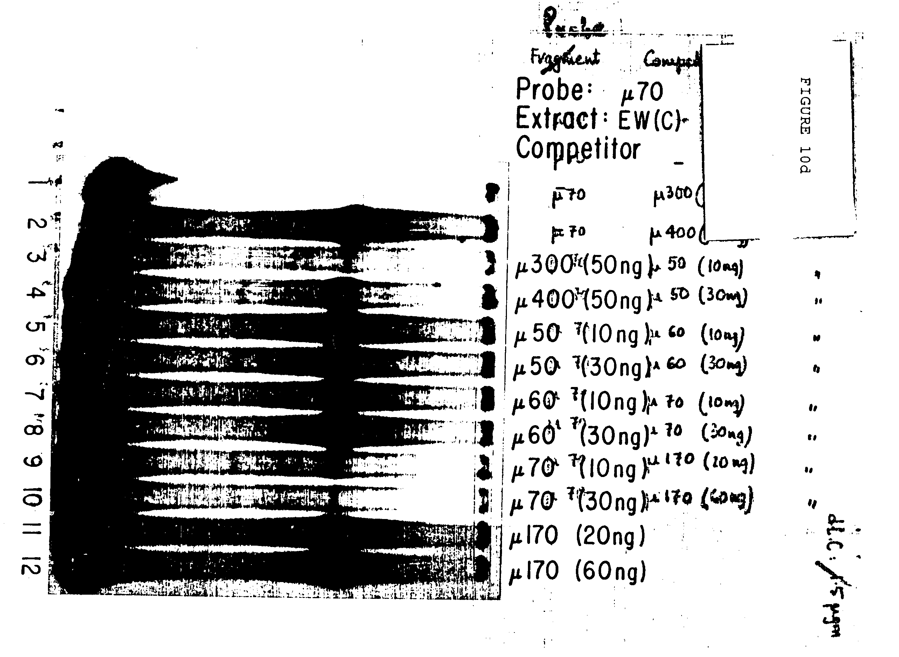

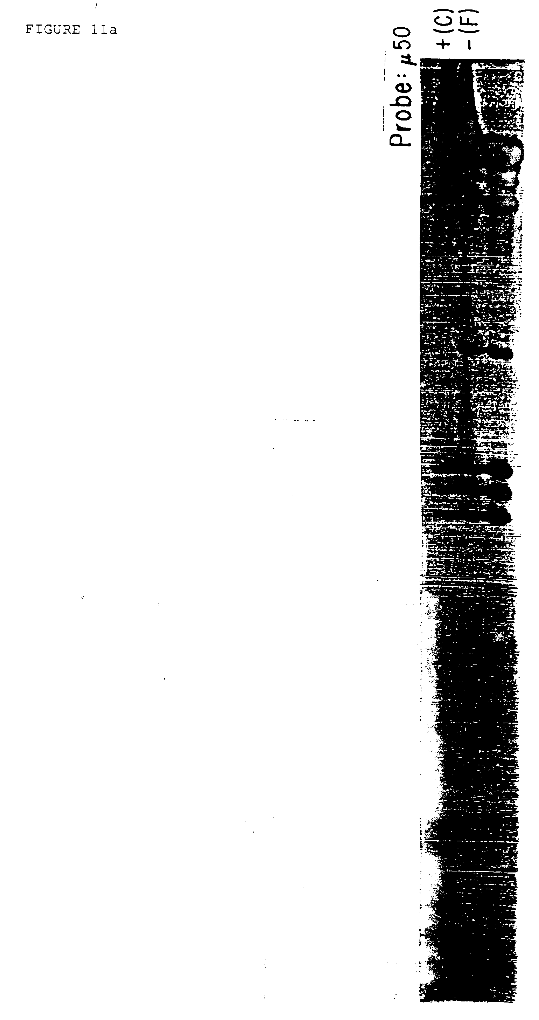

The result of carrying out such an interference experiment using nuclear extracts and on the u50 DNA fragment shows that the complex observed arises via interaction of the IgNF-A protein at the conserved octameric sequence (Figure 3a). The free fragment generates a characteristic G ladder (Figure 11a), lane 2,3) and the complex form (lane 1) is specifically depleted in DNA molecules carrying a methyl group at the G residue indicated by the asterisk which lies in the middle of the conserved octamer. Presumably, modification at this residue seriously impedes the formation of a stable complex between the protein and its cognate sequence. This residue was also shown to be protected against methylation of DMS in vivo. Interestingly, however, none of the other G residues observed to be protected in vivo in this region of the u enhancers appear to be affected in our in vitro interference experiment. Therefore, if these protections in vivo are due to the binding of a protein, this factor is different from IgNF-1 and is not binding to fragment in vitro.

On the u70 fragment several G residues were identified as being important in forming intimate contacts with the binding protein (E) (Figure 11b). On the coding strand bands the 3 G's are significantly reduced in intensity in the complex as compared to the free DNA fragment (Figure 11b, compare lanes 1,2), and on the non-coding strand 2, G's are significantly affected (Figure 11b, compare lanes 3 and 4).

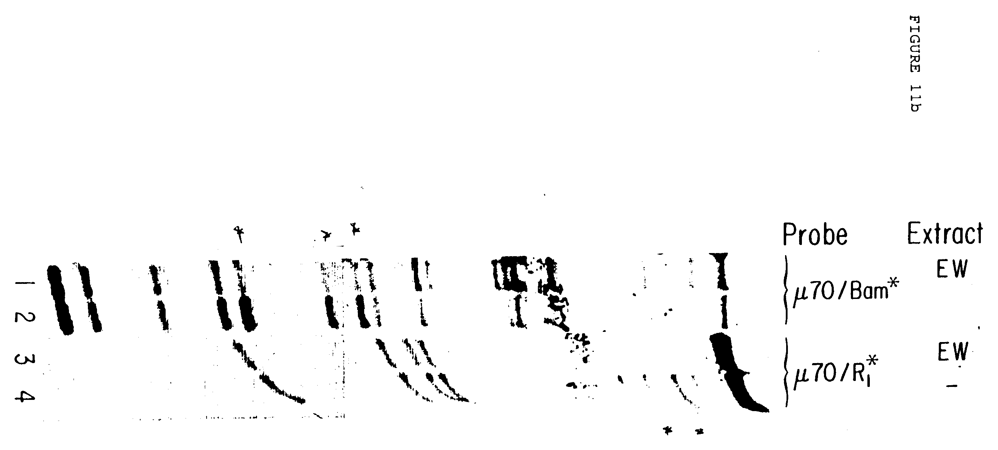

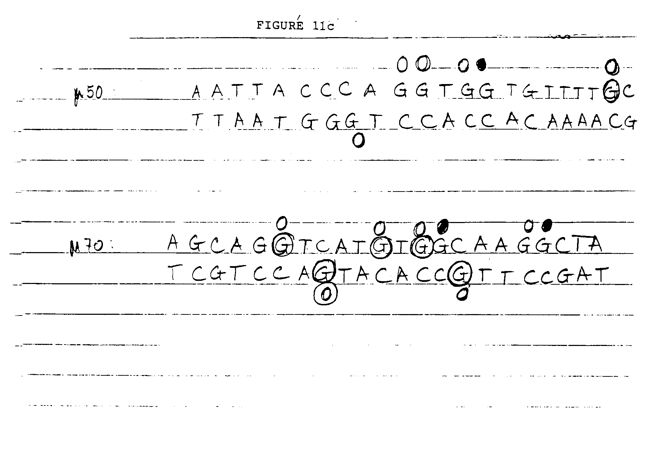

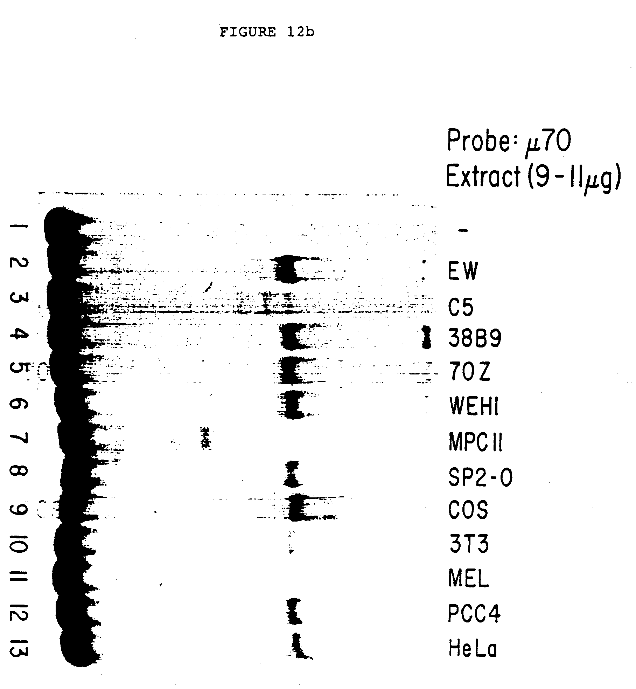

The results of both the in vivo DMS protection experiment and our in vitro methylation interference experiments are summarized in Figure 3c. The open and closed circles above the sequence were the residues identified by Ephrussi et al. to be protected against methylation in vivo whereas the encircled G's are the ones identified by us in vitro. The pattern of protection and interference on the u70 fragment over the consensus sequence is strikingly similar in vivo and in vitro, which indicated strongly that the protein identified here by means of the gel bind assay is the one that interacts with this sequence in vivo. Analogous to u50, however, the second set of protections seen in this region in vivo was not observed in vitro. Tissue specificity of the factors detected: In order to determined whether the proteins identified are limited to expression only in B cells, a large number of extracts made from B cells and non-B cells were screened (Figure 12). Complexes that comigrate with the ones generated and characterized (by competition and methylation interference experiments) in the B cell line EW, were observed on both the fragments u50 and u70 (Figure 12; u 70) in all the cell lines examined. Although the complex generated in each extract has not been further characterized, we interpret this data as indicating that both these factors are non-tissue specific. A second complex (IgNF-B) was observed with the u50 fragment that was restricted to B and T cells only.

A point to note is that although the amount of protein in each lane has been held constant at between 9 and 11 ug, the extent of complex generated was found to vary considerably from extract to extract. Thus showing that quantitive estimations of the abundance of proteins in different cell lines using this assay if not very meaningful at this stage. (This is presumably due to subtle variations in the state of the cells and the extraction conditions for the different cell lines).

In summary, our current analysis of the functional 300 bp PstI-EcoRI fragment of the u enhancer reveals that:
(i) at least 2 different proteins bind within this DNA sequence. One protein (IgNF-A) interacts with an octamer sequence (ATTTGCAT) that is highly conserved upstream of all heavy and light chain variable region genes and is also found in the u enhancer. The second protein interacts with a sequence shown by Ephrussi and Church to be protected in a tissue specific manner against methylation by DMS in whole cells;
(ii) both factors can be detected in nuclear extracts from a variety of cell lines and are therefore not B-cell specific;
(iii) both E1 and E4 sequences hardly compete for the binding of the factor to u70 (which corresponds to E3) thus implying that these sequences do not interact with the same factor, although the sequence homology amongst the sites would have lead one to expect that they should.

### V. Identification of Factors binding to Kappa-light chain enhancer

An enhancer element has also been identified in the major intron of the K light chain gene. Picard and Schaffner showed that the enhancement activity can be localized to a 500 bp AluI-AluI fragment and Queen and Stafford have further refined the 5' and 3' boundaries so that the enhancer may be considered restricted to 275 base pairs within the larger fragment. We have dissected this region into a number of smaller fragments and assayed each of these by means of the gel binding assay for the location of protein binding sites.

A restriction map of the relevant region of the K enhancer is shown in Figure 13a. The black boxes represent sequences identified by Church et al. to be homologous to the putative protein binding domains detected in the u enhancer in vivo.

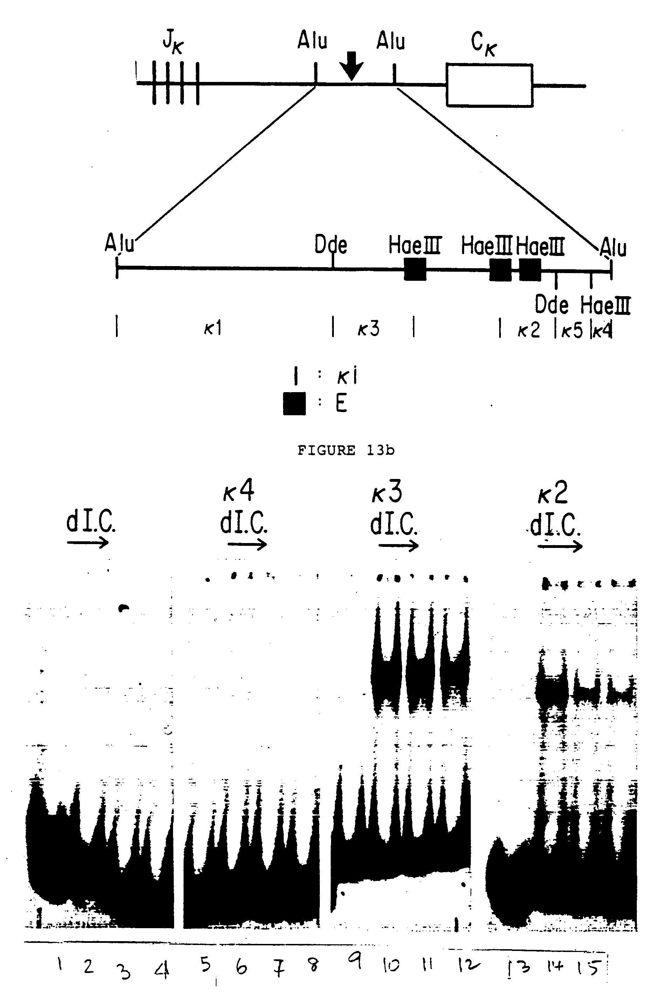

Fragments generated by cutting with Dde and HaeIII (K1, K2, K3, K4 and K5; Figure 13a) were assayed for binding in the presence of increasing amounts of poly(dI.dC).(dI.dc) as a non-specific carrier, K4 and K5 appeared to be obviously negative (Figure 13b, lanes 1-8) while K3 and K2 appeared to be positive (Figure 13b, lanes 10-12 and 14-16). Preliminary results show that the internal fragment does not contain any specific binding sites either. The nucleoprotein complex bands generated with 0.5-1 ng of radiolabelled probe could be detected even in the presence of 3 ug of the carrier (lanes 12, 16, Figure 13b).

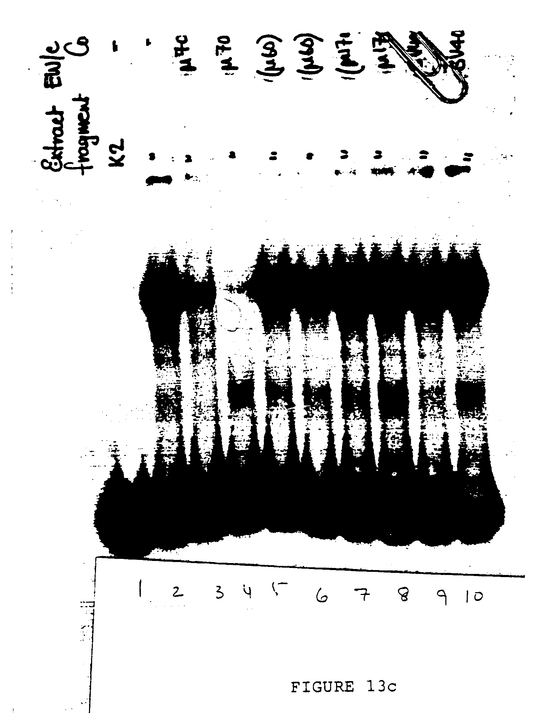

To show that the bands detected represented a specific interaction between a protein and DNA we carried out competition experiments (Figure 13c and 13d). The competition pattern for K2 was strikingly similar to what had been earlier observed with the u70 fragment; this relatively large amounts of u400, the Moloney leukemia virus enhancer, the SV40 enhancer or the K promoter (containing the conserved octa) to k2 did not compete for binding, however u300 and the K enhancer did (data not shown). Since K2 contains a putative E box identified by sequence comparison (as does u70) we competed its binding with smaller fragments from u300 (Figure 5C). The complex is specifically competed away by the addition of unlabelled u70 during the incubation (compare lanes 3 and 4 with lane 2), but not by u60 (lanes 5,6), u170 (lanes 7,8) or the SV40 enhancer (lanes 9,10). Further, the protein that binds to this sequence co-fractionates with the u70 binding activity through two sequential chromatographic steps (Heparin agarose and DEAE Sepharose). Thus we conclude that the same sequence specific protein binds to both the fragments u70 and K2 and therefore there is at least one common protein interacting with both the u and the K enhancers.

The K3 complex (indicated by the arrowhead, Figure 13d) failed to be competed away by u300 (compare lanes 3 and 4 with lane 2), u400 (compare lanes 5 and 6 with lane 2) as a K promoter containing fragment (compare lanes 7 and 8 with lane 2). However, the complex was specifically competed away with both the complete K enhancer (lanes 9,10) and the SV40 enhancer (lanes 11,12). The band below the major K3 complex was seen at variable intensities in different experiments and failed to compete even with the complete K enhancer in this experiment and has not been further investigated at this stage. The observation that the SV40 enhancer specifically competes for binding of this factor is not altogether surprising since this fragment and the SV40 enhancer share an identical stretch of 11 nucleotides.

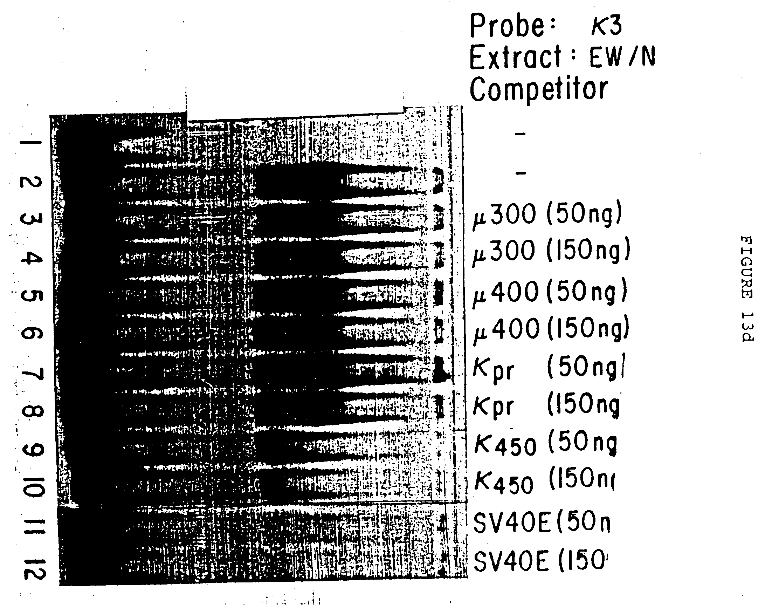

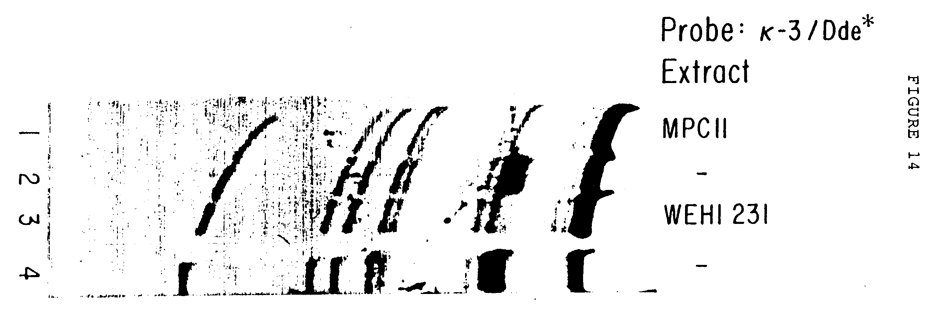

The binding site of this factor on the K3 fragment was localized methylation interference experiments. In two different extracts methylation at three of a stretch of 4 residues within this sequence completely abolished binding (Figure 14, compare lane 1 [complex] and 2 [free]; and lanes 3 [complex] and 4 [free]). This stretch of G's form a part of the conserved region (GGGGACTTTCC) between the SV40 enhancer and K3. Thus the binding site was localized towards one end of the K3 fragment. The results also served to explain the specific competition observed earlier with the SV40 Enhancer. Interestingly, deletion mapping of the K enhancer shows that sequences within the K3 fragment are extremely important for enhancer function.

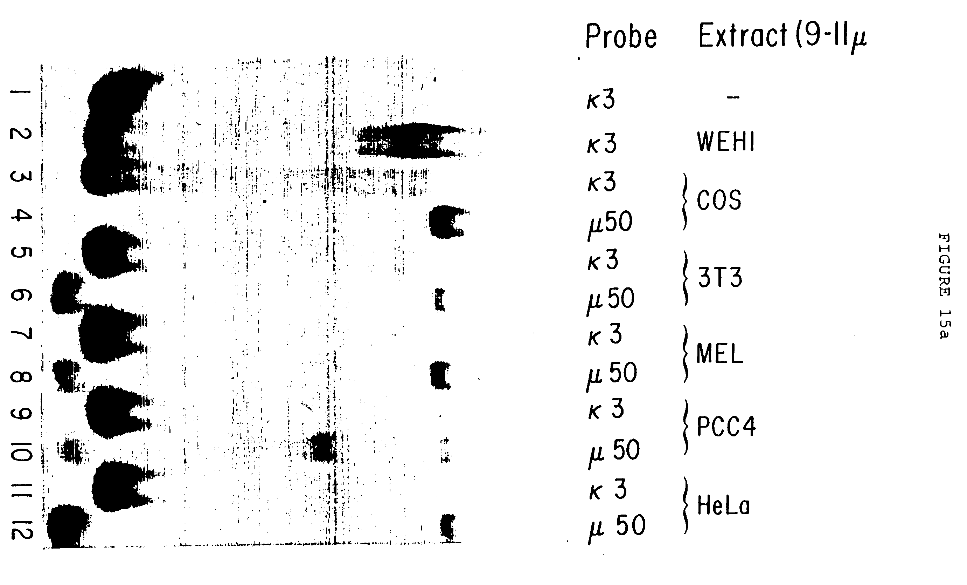

The tissue range of this factor was examined by carrying out binding analysis with K3 in extracts from a variety of cell lines. Nucleoprotein complex formation K3 was detected in a mouse B cell line (Figure 15a, lane 2) but not in 5 other non-B cell lines (Figure 15a, odd numbered lanes from 5-11). Even numbered lanes show that the ubiquitous factor detected by u50 is present in all these cell lines and served as a positive control for the experiment. The factor Kappa 3 therefore appears to be restricted to expression to B lymphoid cells.

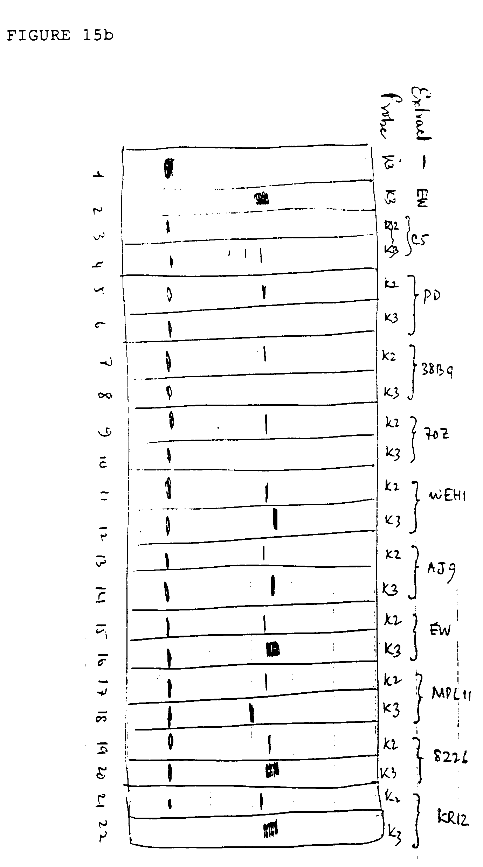

We then examined extracts made from cells at various stages of B cell differentiation (Figure 15B). Interestingly K3 binding protein can be detected in the Abelson murine leukemia virus transformed pre-B cell line PD, in two mouse B cell lines (WEH1231 and AJ9, Figure 15B, lanes 12,14), one human B cell line (EW, Figure 15B, lane 16) mouse myeloma line (MPC22, Figure 15B, lane 18) and 2 human myelomas (KR12 and 8226, Figure 15B, lanes 20,22). However it does not appear to be present in a pre-preB cell line (C5, Figure 15B, lane 4) and in mouse pre-B cell lines (HATFL, 38B9, 70Z, Figure 15B, lanes 6,8,10). Thus this factor appears to be not only tissue-specific, i.e., limited to cells of the B lymphoid lineage, but is also stage-specific within that lineage. In the series of extracts examined, the presence of this factor bears a striking correlation with K expression.

The results with the Kappa enhancer as can be summarized follows: Dissection of the K enhancer enabled to detection of two distinct binding proteins with this DNA. One of these proteins appears to be ubiquitous and interacts with the u heavy chain enhancer as well. The second protein appears to be highly expressed in a stage-specific manner within the B cell lineage and can be detected only in those cell lines where the endogenous K gene is active. There does not appear to be a binding site for this factor in the heavy chain enhancer although there is one in the SV40 enhancer.

## Claims

1. B-cell specific nuclear protein which binds, in a sequence specific manner, to transcriptional regulatory DNA elements of both immunoglobulin light and heavy chain genes.

2. A B-cell nuclear protein of claim 1, which:
a. binds to DNA sequences in the upstream region of both mouse heavy and Kappa light chain gene promoters; and
b. binds to DNA sequences of mouse heavy chain gene enhancer.

3. A B-cell specific nuclear protein of claim 2, wherein the DNA sequences comprise the conserved nucleotide sequence ATTTGCAT.

4. A nuclear protein which binds to enhancer DNA sequences of mouse Kappa light chain.

5. A nuclear protein of claim 4, wherein the enhancer DNA sequences are contained in a 500 bp AluI/AluI DNA fragment.

## Patentansprüche

1. B-Zellen-spezifisches-Zellkernprotein, das in einer sequenzspezifischen Weise an transkriptionale regulatorische DNA-Elemente der Immunglobulin-leichte- und -schwere-Kette-Gene bindet.

2. Ein B-Zellen-Zellkernprotein nach Anspruch 1, das
a. an DNA-Sequenzen in upstream-Region von Promotoren der Maus-schwere- und kappa-leichte-Kette-Gene bindet und
b. an DNA-Sequenzen des Enhancers der Maus-schwere-Kette-Gene bindet.

3. Ein B-Zellen-spezifisches-Zellkernprotein nach Anspruch 2, bei dem die DNA-Sequenzen die konstante Nukleotidsequenz ATTTGCAT aufweisen.

4. Ein Zellkernprotein, das an Enhancer-DNA-Sequenzen der Maus-kappa-leichte-Kette (-DNA) bindet.

5. Ein Zellkernprotein nach Anspruch 4, bei dem sich die Enhancer-DNA-Sequenzen in einem 500 bp AluI/AluI-DNA-Fragment befinden.

## Revendications

1. Protéine nucléaire spécifique des lymphocytes B qui se lie. d'une manière spécifique quant à la séquence. à des éléments d'ADN régulateurs de transcription de gènes de chaîne légère et de chaîne lourde d'immunoglobuline.

2. Protéine nucléaire de lymphocytes B selon la revendication 1. qui
a. se lie à des séquences d'ADN dans la région amont des promoteurs de gènes de chaîne lourde et de chaîne légère kappa de souris; et
b. se lie à des séquences d'ADN de l'amplificateur de gène de chaîne lourde de souris.

3. Protéine nucléaire spécifique des lymphocytes B selon la revendication 2. dans laquelle les séquences d'ADN comprennent la séquence de nucléotides ATTTGCAT conservée.

4. Protéine nucléaire qui se lie aux séquences d'ADN amplificatrices de la chaîne lègère kappa de souris.

5. Protéine nucleaire selon la revendication 4, dans laquelle les séquences d'ADN amplificatrices sont contenues dans un fragment d'ADN de 500 paires de bases AluI/AluI.
